# EUROPEAN PATENT APPLICATION

(11) **EP 4 721 732 A1**
(43) Date of publication of application: **08.04.2026**
(21) Application number: 24815884.2
(22) Date of filing: 31.05.2024
(51) Int. Cl.: A61K 9/20, A61K 31/606, A61P 25/28, A61P 29/00, A61P 25/22, A61P 25/24

(54) **PHARMACEUTICAL COMPOSITION CONTAINING CRISDESALAZINE WITH IMPROVED STABILITY AND DISSOLUTION RATE**

(30) Priority: 02.06.2023 KR 20230071943
(71) Applicant: GNT Pharma Co., Ltd., Yongin-si, Gyeonggi-do 17096 (KR)
(72) Inventor: GWAG, Byoung Joo, Yongin-si, Gyeonggi-do 16951 (KR); CHO, Sung Ig, Seoul 06090 (KR); AZAM, Sharif Mohammed Shafioul, Pyeongtaek-si, Gyeonggi-do 17882 (KR); JIN, Jing Yu, Suwon-si, Gyeonggi-do 16708 (KR); AN, Chun San, Suwon-si, Gyeonggi-do 16682 (KR); PARK, Young-Joon, Seoul 06583 (KR); CHOI, Sook, Seoul 06583 (KR); HWANG, Sung-Joo, Seongnam-si, Gyeonggi-do 13644 (KR)
(74) Representative: Maiwald GmbH
(86) International application number: PCT/KR2024/007420
(87) International publication number: WO 2024/248510

(57) **Abstract**

The present invention relates to a pharmaceutical composition comprising crisdesalazine or a salt thereof, which has improved stability by reducing the content of related substances of crisdesalazine, and has an increased dissolution rate by improving the intrinsic solubility thereof. The composition of the present invention secures stability and bioavailability, and thus is more effective in treating neurodegenerative diseases (Alzheimer's disease, Parkinson's disease, amyotrophic lateral sclerosis, Huntington's disease, epilepsy accompanied by free radical neurotoxicity, cerebral trauma, spinal injury, and the like), inflammatory diseases (gastritis, colitis, pancreatitis, arthritis, diabetic inflammation, inflammatory bowel disease, nephritis, hepatitis, arteriosclerosis inflammation, and the like), stress disorders (anxiety disorder, depression, and the like), and the like.

## Description

### TECHNICAL FIELD

This application is based on and claims priority from Korean Patent Application No. 10-2023-0071943, filed on 2 June 2023, the disclosure of which is incorporated herein in its entirety by reference.

The present invention relates to a pharmaceutic or pharmaceutical composition comprising crisdesalazine or a pharmaceutically acceptable salt thereof as an active ingredient, and particularly to a pharmaceutical composition having improved stability, dissolution rate, bioavailability, etc.

### BACKGROUND ART

Crisdesalazine (2-hydroxy-5-[2-(4-trifluoromethylphenyl)ethylamino] benzoic acid) acts as both an antioxidant and an anti-inflammatory agent through selective inhibition of microsomal prostaglandin E₂ synthase (mPGES-1), the terminal enzyme in the prostaglandin E₂ (PGE₂) pathway induced by proinflammatory cytokines. Additionally, crisdesalazine is known to be effective in the treatment of various neurodegenerative, inflammatory, and psychiatric disorders.

PCT Patent Publication WO 01/79153 Al provides a pharmaceutical composition comprising crisdesalazine or a salt thereof for protecting the central nerves from acute or chronic damage to the central nervous system, and also provides a method for treating or preventing a nervous system disease associated with NMDA (N-methyl-D-aspartate) neurotoxicity, Zn²⁺ neurotoxicity or oxidative stress, comprising administering a therapeutically effective amount of the composition to a patient or animal suffering from acute or chronic damage to the central nervous system. Specifically, crisdesalazine or its salts have been reported to have the function of protecting the central nervous system from damage that causes ischemia, hypoxia, hypoglycemia, traumatic brain injury, traumatic spinal cord injury, epilepsy, Huntington's disease, Parkinson's disease, Alzheimer's disease, or amyotrophic lateral sclerosis.

PCT Patent Publication WO 2007/119973 and US Patent No. 8,455,470 B2 disclose that crisdesalazine or its salts are effective for treating one or more inflammatory diseases selected from the group consisting of gastritis, colitis, pancreatitis, arthritis, diabetic inflammation, inflammatory bowel disease, nephritis, hepatitis, and inflammation associated with arteriosclerosis. PCT Patent Publication WO2010-090494 A2 discloses that crisdesalazine or its salts are effective for treating or preventing stress disorders, anxiety disorders, and depression.

Experimentally, the inventors have confirmed that crisdesalazine is a very poorly soluble drug, exhibiting low solubility under acidic pH conditions and high solubility under alkaline pH conditions. If a formulation is formulated without considering these physicochemical properties, the drug will not dissolve in the gastrointestinal tract after oral administration, resulting in poor absorption rates, making it difficult to achieve an effective therapeutic effect. Moreover, when alkaline ingredients are included in formulations to enhance solubility, they can adversely affect the stability of crisdesalazine. Therefore, obtaining a formulation that satisfies both stability and dissolution rate for crisdesalazine presents a challenge.

### DISCLOSURE

### Technical Problem

Therefore, the problem to be solved by the present invention is to provide a pharmaceutical composition having improved stability while improving the dissolution rate of crisdesalazine or a pharmaceutically acceptable salt thereof, which is a poorly water-soluble drug. That is, the present invention aims to provide an oral pharmaceutical formulation in which crisdesalazine, which is poorly water-soluble, is improved to have better solubility and absorbability in the gastrointestinal tract, and at the same time, to provide a pharmaceutical product in which the long-term stability of the formulation, particularly the long-term stability of the impurities, is secured.

### Technical Solution

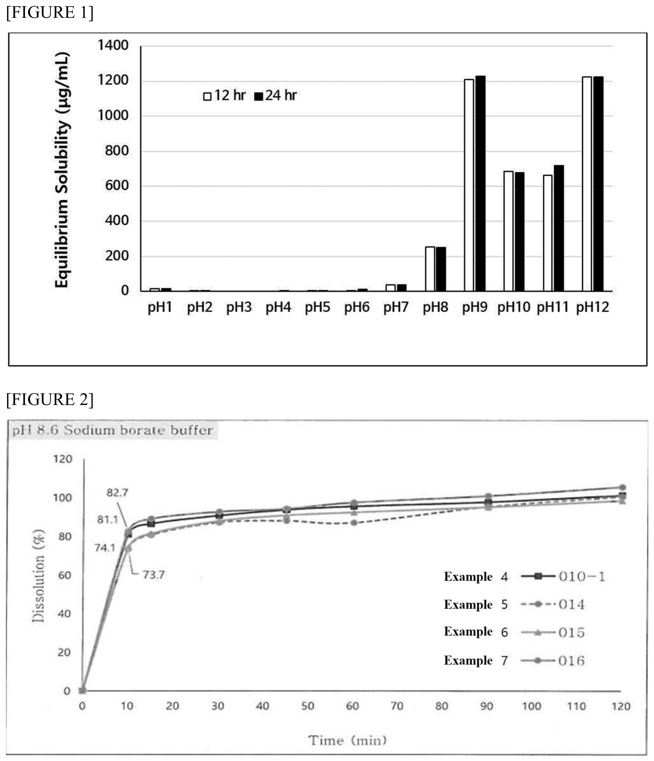

First, the inventors of the present invention measured the pH-dependent equilibrium solubility of crisdesalazine (2-hydroxy-5-[2-(4-trifluoromethylphenyl)ethylamino]benzoic acid), and the results of the solubility at 12 hours and 24 hours were as shown in Table 1 and Figure 1 below. As shown in the table below, the equilibrium solubility of crisdesalazine in purified water was very low at pH 7 or lower (38.2 µg/mL or less at 12 hours and 39.7 µg/mL or less at 24 hours), but the solubility increased significantly at pH 8 (253.9 µg/mL at 12 hours and 251.6 µg/mL at 24 hours), and showed a greater pH-dependent increase at higher pHs.

**[Table 1]**

| pH of the solution | Equilibrium Solubility (µg/mL) | |
|---|---|---|
| | 12 hours | 24 hours |
| 1 | 16.4 | 15.7 |
| 2 | 2.6 | 1.7 |
| 3 | BLOD | BLOD |
| 4 | BLOD | 2.2 |
| 5 | 0.7 | 5.9 |
| 6 | 6.4 | 11.5 |
| 7 | 38.2 | 39.7 |
| 8 | 253.9 | 251.6 |
| 9 | 1210.0 | 1229.1 |
| 10 | 685.0 | 677.7 |
| 11 | 662.2 | 717.9 |
| 12 | 1225.0 | 1224.7 |
| BLOD: Below of Limit of Detection | | |

As shown in the results in the table above and Figure 1, solubility increases at high pH, and thus, it was thought that using an alkalizing agent could enhance solubility and bioavailability. However, increasing the pH within the composition negatively affected stability, and consequently, achieving both stability and dissolution rate was challenging.

Specifically, to achieve the dual goals of stability and dissolution rate (bioavailability), one embodiment of the present invention provides a pharmaceutical composition comprising crisdesalazine or a pharmaceutically acceptable salt thereof as an active ingredient and a surfactant having an HLB (Hydrophilic and Lipophilic Balance) value of 8 or greater.

The inventors evaluated various additives, and among them, anionic surfactants and nonionic surfactants with an HLB value of 8 or higher were the most preferable. Cationic surfactants have no significant difference in solubilizing ability compared to the solubility of crisdesalazine itself, and are unsuitable as solubilizers when contained in formulations with crisdesalazine because the amount of related substances, which are impurities, increases under accelerated conditions. Furthermore, nonionic surfactants with an HLB value of less than 8 showed little effect in improving drug solubility even in aqueous solutions containing more than 10%. On the other hand, the inclusion of anionic surfactants and nonionic surfactants with an HLB value of 8 or higher improved solubility by more than twofold, resulting in an increase in the dissolution rate of the formulation.

Crisdesalazine or its salt has the function of protecting the central nervous system damage that causes ischemic nerve damage, hypoxia, hypoglycemia, brain trauma, spinal trauma, epilepsy, Huntington's disease, Parkinson's disease, Alzheimer's disease or amyotrophic lateral sclerosis. It is preferable that the content of these in the formulation contain 10-500 mg, and more preferably 50-200 mg. However, in the case of a formulation exceeding 200 mg, the size of the formulation may be large and inconvenient to take, but when such a surfactant is used, it is possible to manufacture a formulation of a size suitable for taking even in a high-content crisdesalazine formulation of 200 mg or more.

As an anionic surfactant used, it is preferable to use, for example, sodium lauryl sulfate, sodium dodecyl sulfate, etc. As a nonionic surfactant of the present invention, a surfactant commonly used in the pharmaceutical field having an HLB value of 8 or higher can be used. For example, polyethylene glycol-15-hydroxystearate (e.g., Solutol^{™} HS 15), polyoxyethylene glycolated natural or hydrogenated castor oil (e.g., Cremophor^{™} RH 40, Cremophor^{™} RH 60), polyoxyethylene-polyoxypropylene copolymers (e.g., Poloxamer^{™} 407, Poloxamer^{™} 118), sucrose fatty acid esters (e.g., RyotoSugar Ester^{™} S-1570, S-1670, P-1570, P-1670, L-1695), synthetic vitamin E derivatives (e.g., vitamin E TPGS^{™}), sorbitan esters (e.g., sorbitan monolaurate) and polyoxyethylene sorbitan fatty acid esters (e.g., Polysorbate^{™} 80, Polysorbate^{™} 20, Polysorbate^{™} 40), fatty acid macrogol glycerides (e.g., Gelucire 44/14^{™}), polyglyceryl fatty acid esters (e.g., Almax-9420, Almax-9640, Plurol oleique MB), lecithin, caprylcaproyl polyoxy-8-glyceride (e.g., Labrasol^{™}), sorbitan fatty acid esters (e.g., sorbitan laurate), etc. can be used.

In a preferred embodiment of the present invention, the surfactant used together with crisdesalazine according to the present invention is sodium lauryl sulfate, which is an anionic surfactant. In a preferred embodiment of the present invention, the surfactant used together with crisdesalazine according to the present invention is a surfactant having an HLB value of 8 or higher among polyethylene glycol-15-hydroxystearate, polyoxyethylene glycolated natural or hydrogenated castor oil, sucrose fatty acid esters (e.g., RyotoSugar Ester^{™} S-1570, S-1670, P-1570, P-1670, L-1695), polyoxyethylene-polyoxypropylene copolymers, synthetic vitamin E derivatives (e.g., vitamin E TPGS^{™}), or polyoxyethylene sorbitan fatty acid esters (e.g., polysorbate 80). In a more preferred embodiment of the present invention, the surfactant used together with crisdesalazine according to the present invention is sodium lauryl sulfate, polmoxamer-407, or a mixture thereof.

The surfactant is preferably used in an amount of 0.1-10 wt% of the total weight of the composition, more preferably 0.5-5 wt%.

In the present disclosure, "pharmaceutically acceptable salt" means a salt formed by a non-toxic or substantially non-toxic acid or base. Pharmaceutically acceptable salts include, but are not limited to, salts prepared with lithium, sodium, potassium, calcium, ammonium, magnesium, or organic amino acids (e.g., diethylamine).

One embodiment of the present invention provides a pharmaceutical composition substantially free of lactose. The inventors conducted "compatibility" evaluation experiments between crisdesalazine and various excipients. The results showed poor compatibility with calcium phosphate dibasic, hydroxypropyl methylcellulose (HPMC), meglumine, magnesium oxide (MgO), etc.

Lactose was judged to be compatible with the formulation in a 13-week compatibility study, with only minor powder coagulation and minimal changes in the amount of the impurities. However, long-term stability testing of actual formulations for 1-2 years or longer revealed that the composition was undesirable. In other words, the long-term stability of crisdesalazine and lactose was difficult to predict based solely on short-term compatibility evaluation tests. Therefore, it is preferable that the composition according to the present invention substantially does not contain lactose, dibasic calcium phosphate, HPMC, meglumine, MgO, etc., and in particular, it is preferable that the composition substantially does not contain lactose.

In the present invention, "substantially free of" means that it is contained in an amount of 2 wt% or less based on the total weight of the composition, preferably it is contained in an amount of 1 wt% or less, more preferably it is contained in an amount of 0.5 wt% or less, and most preferably it is contained in an amount of not at all.

Another embodiment of the present invention provides a pharmaceutical composition comprising mannitol, microcrystalline cellulose, or a mixture thereof as a diluent instead of lactose.

"Diluent" refers to an inert substance used as a filler to create desirable bulk, flow properties, and compression characteristics in the manufacture of solid dosage forms. Examples thereof include mannitol, maltitol, isomalt, sucrose, lactose, colloidal silicon dioxide, dextrin, dextrates, microcrystalline cellulose, crystalline cellulose, glucose, polydextrose, starch, pregelatinized starch, corn starch, potato starch, cellactose, anhydrous lactose, and mixtures thereof. The present inventors have determined that among these various diluents, mannitol and/or microcrystalline cellulose are preferred. Therefore, one embodiment of the present invention provides a composition comprising mannitol or microcrystalline cellulose as a diluent. Another preferred embodiment of the present invention provides a composition comprising both mannitol and microcrystalline cellulose as diluents. In the present invention, the mannitol may be D-mannitol, for example, 100SD and/or 200SD.

The content of mannitol and/or microcrystalline cellulose may range from 5 to 80 wt% of the total weight of the composition, with 10 to 60 wt% being preferred.

One embodiment of the present invention provides a pharmaceutical composition comprising 5-70 wt% of crisdesalazine or a pharmaceutically acceptable salt thereof, 5-80 wt% of mannitol, 5-80 wt% of microcrystalline cellulose, 2-10 wt% of disintegrant(s), 0.1-10 wt% of sodium lauryl sulfate, and 0.1-10 wt% of lubricant(s), relative to the total weight of the composition, and preferably, a pharmaceutical composition in the form of a tablet comprising 5-50 wt% of crisdesalazine or a pharmaceutically acceptable salt thereof, 10-60 wt% of mannitol, 10-60 wt% of microcrystalline cellulose, 2-10 wt% of disintegrant(s), 0.5-5 wt% of sodium lauryl sulfate, and 0.5-5 wt% of lubricant(s), relative to the total weight of the composition.

In the composition of the present invention, disintegrant(s) such as carboxymethylcellulose calcium (CMC-Ca), crospovidone, sodium starch glycolate, croscarmellose sodium, and low-substituted hydroxypropyl cellulose may be used. In a preferred embodiment of the present invention, the disintegrant is croscarmellose sodium.

In the composition of the present invention, lubricant(s) such as colloidal silica, magnesium stearate, stearic acid, talc, sodium stearyl fumarate, sodium stearyl fumarate, Ryoto^{™} ester, and hydrogenated castor oil may be used. In a preferred embodiment of the present invention, the lubricant is sodium stearyl fumarate. The content of the lubricant may range from 0.1 to 5 wt% based on the total weight of the composition.

The composition of the present invention is in tablet form, and such tablets can be coated with coating materials well known to those skilled in the art. Commercially available coating products can be used as such coating materials. For example, coating materials such as Colorcon's Opadry^{®} 03B62519, 03B59005, 85F530135, and 85F220008 can be used. These coating materials can be used alone, mixed as a coating material, or sequentially coated. For example, the tablet of the present invention is a double-coated tablet that is sealed after subcoating.

In a preferred embodiment of the present invention, the preferred maximum range of use of crisdesalazine and excipients (but not limited to the materials listed below) is as shown in Table 2 below.

**[Table 2]**

| Intended Use | Ingredient | Example 2 (200 mg) | Desired Use Ragne (mg) | Use Range (%) | Maximum Use Range (%) |
|---|---|---|---|---|---|
| Active ingredient | Crisdesalazine | 200 | 50-200 | 5-50 | 5 ~70 |
| Diluent | D-Mannitol | 97 | 42-252 | 10-60 | 5-80 |
| Diluent | Microcrystalline cellulose | 80 | 42-252 | 10-60 | 5-80 |
| Disintegrant | Croscarmellose sodium | 15 | 8.4-42 | 2-10 | 2-10 |
| Solubilizer | Sodium lauryl sulfate | 4 | 2.1-21 | 0.5-5 | 0.1-10 |
| Lubricant | Sodium stearyl fumarate | 4 | 2.1-21 | 0.5-5 | 0.1-10 |
| Coating Material | Opadry (e.g., 03B590005 and 85F220008) | 20 | 12.6-42 | 3-10 | 3-15 |
| Average (weight/composition) | | 420 mg | Composition | 100% | 100% |

One embodiment of the present invention also provides a pharmaceutical composition comprising crisdesalazine or a pharmaceutically acceptable salt thereof as an active ingredient, wherein the pH is less than 7 when dispersed in 250 mL of purified water.

Considering the expected human therapeutic dose of crisdesalazine, the maximum (individual) related substance should be less than 0.2% according to the ICH Q3B(R2) guideline. When formulating a formulation using an alkaline pH excipient, the formation of related substances, which are impurities, increases and the stability of the drug within the formulation decreases. To overcome this problem, a formulation can be prepared by excluding alkaline excipients and additives. In other words, by maintaining the pH of the formulation below 7, the stability of the formulation can be maintained.

Therefore, one embodiment of the present invention provides a pharmaceutical composition containing crisdesalazine or a pharmaceutically acceptable salt thereof, substantially free of alkaline additives such as sodium carbonate, sodium bicarbonate, sodium hydroxide, potassium hydroxide, magnesium hydroxide, magnesium carbonate, calcium phosphate, sodium phosphate, dibasic calcium phosphate, or magnesium trisilicate.

To maintain the pH of the formulation below 7, the present invention provides a pharmaceutical composition comprising (i) crisdesalazine or a pharmaceutically acceptable salt thereof and (ii) an acidifying agent, and further comprising (iii) a surfactant having an HLB of 8 or higher to ensure the dissolution rate of the crisdesalazine component, which is poorly soluble in water. The surfactant having an HLB of 8 or higher may be an anionic surfactant and/or a nonionic surfactant, as described above.

Examples of the acidifying agent include propionic acid, isobutylic acid, oxalic acid, malic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, hydrochloric acid, bromic acid, nitric acid, carbonic acid, monohydrogen-carbonic acid, phosphoric acid, monohydrogen-phosphoric acid, dihydrogen-phosphoric acid, sulfuric acid, monohydrogen-sulfuric acid, hydrogen iodide, phosphorous acid, glucuronic acid, galactunoric acid, etc. can be used. In a preferred embodiment of the present invention, the acidifying agent is fumaric acid, succinic acid, tartaric acid, oxalic acid, or a mixture thereof.

In addition to the aforementioned ingredients, the composition of the present invention may further contain additional binders, disintegrants, lubricants, dispersants, etc. suited to the formulation characteristics (within the scope that does not impede the purpose of the present invention), and may be provided in the form of oral preparations such as tablets, capsules, granules, and powders. Among these, the most preferred formulations are tablets and capsules. These formulations may be manufactured according to methods commonly used in the pharmaceutical field. For example, they may be manufactured in various ways using conventional tablet or capsule manufacturing methods such as direct mixing, wet granulation, and dry granulation, and there are no particular limitations on the manufacturing method.

In the present invention, the term "binder" refers to a substance capable of imparting elasticity and adhesiveness to increase the strength of the formed formulation, particularly the strength of the tablet. For example, the binder may be, but is not limited to, polyvinylpyrrolidone (PVP), carboxymethylcellulose-sodium (CMC-Na), starch paste, gelatin, hydroxymethylcellulose, xanthan gum, gum arabic, and hydroxypropylcellulose (HPC).

The pharmaceutical composition according to the present invention can be used for the treatment, improvement or prevention of neurodegenerative diseases (Alzheimer's disease, Parkinson's disease, amyotrophic lateral sclerosis, Huntington's disease, epilepsy accompanied by free radical neurotoxicity, brain trauma and spinal cord injury, etc.), inflammatory diseases (gastritis, colitis, pancreatitis, arthritis, diabetic inflammation, inflammatory bowel disease, nephritis, hepatitis, arteriosclerotic inflammation, etc.), stress disorders (anxiety disorder, depression, etc.), etc., but the present invention is not limited to such uses.

### Advantageous Effects

The present invention provides a pharmaceutical composition comprising crisdesalazine or a pharmaceutically acceptable salt thereof as an active ingredient, which has secured stability and a good dissolution rate, thereby ensuring bioavailability.

### DESCRIPTION OF DRAWINGS

Figure 1 shows the results of a pH-dependent equilibrium solubility experiment of crisdesalazine in a buffer solution at 1 and 12 hours.
Figure 2 shows the results of a 120-minute dissolution rate evaluation of Examples 4-7 according to the present invention. A sodium borate buffer with a pH of 8.6 was used as the dissolution medium.

### MODE FOR DISCLOSURE

Hereinafter, the present invention will be described in detail, using examples and the like, to aid understanding. However, the examples according to the present invention may be modified in various different forms, and the scope of the present invention should not be construed as being limited to the following examples. The examples of the present invention are provided to more fully explain the present invention to those of average skill in the art.

### Preparation of Example 1-3

Tablets containing crisdesalazine as the main ingredient were prepared according to the composition shown in the table below. Specifically, crisdesalazine, croscarmellose sodium, and sodium lauryl sulfate were mixed, sieved (using a 20 mesh), and then microcrystalline cellulose and D-mannitol were added. A lubricant (sodium stearyl fumarate) sieved through a 40 mesh was added to this mixture, and the tablets were compressed into round tablets using a rotary tablet press. Film coating was then performed using Opadry.

Dosage-Proportional Pharmaceutical Composition of 2-Hydroxy-5-[2-(4-Trifluoromethylphenyl)ethylamino]benzoic Acid (Crisdesalazine)

**[Table 3]**

| Intended Use | Ingredient | Example 1 | Example 2 | Example 3 |
|---|---|---|---|---|
| Active ingredient | Crisdesalazine | 100 | 200 | 50 |
| Diluent | D-Mannitol | 48.5 | 97 | 24.25 |
| Diluent | Microcrystalline cellulose | 40 | 80 | 20 |
| Disintegrant | Croscarmellose sodium | 7.5 | 15 | 3.75 |
| Solubilizer | Sodium lauryl sulfate | 2 | 4 | 1 |
| Lubricant | Sodium stearyl fumarate | 2 | 4 | 1 |
| Coating Material | Opadry 03B590005 | 4 | 8 | 2 |
| Coating Material | Opadry 85F220008 | 6 | 12 | 3 |
| Average Weight (mg/formulation) | | 210 mg | 420 mg | 105 mg |

### Evaluation of Hardness, Friability, etc. of Example 1-3

The tablets manufactured in Example 1-3 were evaluated, and the results are shown in the table below.

**[Table 4]**

| Intended Use | Specifications / Raw Material | Example 1 | Example 2 | Example 3 |
|---|---|---|---|---|
| Active ingredient | Crisdesalazine | 100 | 200 | 50 |
| Weight change (core) | Targeted weight ± 3% | 200 | 400 | 100 |
| Hardness (KP) | Range | 12 ~ 20 | 12 ~ 20 | 3 ~ 8 |
| Thickness (mm) | Range | 2.7 ~ 4.7 | 3.7 ~ 5.7 | 2.3 ~ 4.3 |
| Punch (mm) | (L x W) or Ø | 10.9 x 5.3 | 13.5 x 7.0 | Ø: 6 |
| Friability (%) | 1.0 or less | less than 1.0 | less than 1.0 | less than 1.0 |
| Weight change (coating) | Targeted weight ± 2% | 210 | 420 | 105 |

As shown in the table above, tablets with good hardness, friability, and other properties were manufactured using the formulations of Examples 1-3.

### Long-Term Stability Evaluation of Examples 1 and 2

Using the same formulations as in Examples 1 and 2, tablets were manufactured through scale-up. Long-term stability was evaluated under conditions of 25±2°C and 60±5% RH (relative humidity). The results are presented in the tables below.

**[Table 5]**

| Evaluation Items | Specifications | Crisdesalazine, 100 mg tablet | | | | | | | |
|---|---|---|---|---|---|---|---|---|---|
| | | Test Period | | | | | | | |
| | | Initial | 1M | 3M | 6M | 9M | 12M | 18M | 24M |
| Dissolution Rate (Average %) | 45 min, 70% or more (Q) | 95.3 | 94.7 | 99.1 | 94.4 | 93.6 | 92.5 | 94.9 | 95.5 |

| Impurity(ies) (%) | | | | | | | | | |
|---|---|---|---|---|---|---|---|---|---|
| RRT 1.3 | 0.2% or less | 0.08% | 0.08% | 0.07% | 0.07% | 0.07% | 0.08% | 0.08% | 0.08% |
| RRT 1.5 | 0.2% or less | 0.06% | 0.06% | 0.05% | 0.06% | 0.06% | 0.06% | 0.05% | 0.05% |
| Maximum unknown impurity | 0.15% or less | 0.02% | 0.02% | 0.02% | 0.02% | 0.02% | 0.02% | 0.03% | 0.02% |
| Total impurities | 0.8% or less | 0.2% | 0.16% | 0.14% | 0.15% | 0.14% | 0.20% | 0.16% | 0.15% |
| Content (%) | 95.0~105.0% | 99.4 | 99.1 | 99.1 | 100.7 | 101 | 101.4 | 102 | 98.4 |

**[Table 6]**

| Evaluation Items | Specifications | Crisdesalazine, 200 mg tablet | | | | | | | |
|---|---|---|---|---|---|---|---|---|---|
| | | Test Period | | | | | | | |
| | | Initial | 1M | 3M | 6M | 9M | 12M | 18M | 24M |
| Dissolution Rate (Average %) | 45 min, 70% or more (Q) | 92.7 | 88.3 | 90.6 | 91.5 | 91.5 | 87.8 | 93.9 | 88.2 |

| Impurity(ies) (%) | | | | | | | | | |
|---|---|---|---|---|---|---|---|---|---|
| RRT 1.3 | 0.2% or less | 0.07% | 0.08% | 0.07% | 0.07% | 0.08% | 0.07% | 0.07% | 0.08% |
| RRT 1.5 | 0.2% or less | 0.06% | 0.06% | 0.05% | 0.06% | 0.06% | 0.06% | 0.06% | 0.05% |
| Maximum unknown impurity | 0.15% or less | 0.02% | 0.02% | 0.02% | 0.02% | 0.02% | 0.03% | 0.03% | 0.02% |
| Total impurities | 0.8% or less | 0.2% | 0.16% | 0.14% | 0.15% | 0.15% | 0.16% | 0.16% | 0.15% |
| Content (%) | 95.0~105.0% | 100.1 | 100.1 | 99.4 | 100.2 | 101.2 | 101.9 | 100.5 | 98.7 |

| | | | | | | | | | |
|---|---|---|---|---|---|---|---|---|---|
| RRT: Relative retention time | | | | | | | | | |

The above stability study data confirms the stability of the composition of the present invention in long-term stability evaluation. Each impurity (the maximum value of known impurity, unknown impurity, and total impurities) remained within the specifications throughout the test period. In other words, the composition of the present invention was confirmed to maintain improved stability and dissolution rate over a long period of time.

For reference, as shown in Figure 1, equilibrium solubility was observed to increase between pH 8 and 9 and to be maintained for up to 12 hours. Therefore, a dissolution test was conducted to study the release profile.

The dissolution test was conducted using a borate buffer solution (pH 8.6, 900 mL) in accordance with the Korean Pharmacopoeia Dissolution Test Method 2 at a rotation speed of 75 rpm. The amount of dissolution was evaluated at 0, 10, 15, 30, 60, 90, and 120 minutes after the start of the test. As an analysis method, the absorbance was measured at a wavelength of 336 nm for the standard solution and test solution according to the ultraviolet-visible absorbance measurement method in the general test methods of the Korean Pharmacopoeia.

### Accelerated Stability Evaluation of Example 2

After scaling up and manufacturing tablets using the same formulation as Example 2, stability was evaluated under conditions of 40±2°C and 75±5% RH. The results are shown in the table below.

**[Table 7]**

| Evaluation Items | Specificati ons | Batch 1 | | | Batch 2 | | | Batch 3 | | |
|---|---|---|---|---|---|---|---|---|---|---|
| | | Test time | | | Test time | | | Test time | | |
| | | Initial | 2M | 3M | Initial | 2M | 3M | Initial | 2M | 3M |
| Minimum Dissolutio n Rate (%) | 45 min, 70% or more (Q) | 94 | 92 | 97 | 95 | 93 | 93 | 91 | 91 | 95 |

| Impurity(ies) (%) | | | | | | | | | | |
|---|---|---|---|---|---|---|---|---|---|---|
| RRT 1.3 | 0.2% or less | BLOQ (0.065 %) | BLOQ (0.051 %) | BLOQ (0.041 %) | BLOQ (0.060 %) | BLOQ (0.052 %) | BLOQ (0.050 %) | BLOQ (0.061 %) | BLOQ (0.049 %) | BLOQ (0.037 %) |
| RRT 1.5 | 0.2% or less | 0.0810 % | 0.0770 % | 0.0750 % | BLOQ (0.077 %) | BLOQ (0.077 %) | BLOQ (0.077 %) | BLOQ (0.077 %) | BLOQ (0.076 %) | BLOQ (0.071 %) |
| Maximum Individual Unknown Impurity | 0.15% or less | BLOQ (0.043 %) | BLOQ (0.046 %) | BLOQ (0.028 %) | BLOQ (0.047 %) | BLOQ (0.046 %) | BLOD (0.027 %) | BLOQ (0.048 %) | BLOQ (0.046 %) | BLOQ (0.028 %) |
| Total Impurities | 0.8% or less | 0.242 % | 0.272 % | 0.189 % | 0.174 % | 0.266 % | 0.202 % | 0.215 % | 0.262 % | 0.190 % |
| Content (%) | 95.0~ 105.0 % | 100 | 101 | 100 | 103 | 101 | 100 | 102 | 103 | 102 |

| | | | | | | | | | | |
|---|---|---|---|---|---|---|---|---|---|---|
| RRT: Relative retention Time, BLOD: Below of LOD (Limit of Detection) [LOD=0.025%], BLOQ: Below of LOQ (Limit of Quantitation) [LOQ=0.08%]. | | | | | | | | | | |

In all three batches manufactured and evaluated, the composition according to the present invention was confirmed to be stable for a sufficient period of time under accelerated conditions. In other words, the composition according to the present invention was confirmed to maintain improved stability and dissolution rate even after storage under accelerated conditions.

### Preparation of Examples 4-7 and Comparative Examples 1-4

Tablets containing crisdesalazine as the active ingredient were manufactured using the compositions shown in the table below. Except for film coating, the tablets were manufactured using a method similar to Example 1. Comparative Examples 1, 2, 3, and 4 were manufactured by simply mixing the ingredients in a capsule formulation and then filling them into empty capsules.

**[Table 8]**

| Intended Use | Ingredient (Unit: wt%) | Exam ple 4 010-1 | Exam ple 5 014 | Exam ple 6 015 | Exam ple 7 016 | Comparat ive Example 1 GNT 200#3 | Comparat ive Example 2 GNT 200#4 | Comparat ive Example 3 GNT 200#1 | Comparat ive Example 4 GNT 200#2 |
|---|---|---|---|---|---|---|---|---|---|
| Active ingredien t | Crisdesalazi ne | 200 | 200 | 200 | 200 | 200 | 200 | 200 | 200 |
| Diluent | D-Mannitol (100SD) | 72 | 75 | 78 | 75 | - | - | - | - |
| | Microcrystal line cellulose | 75 | 75 | 75 | 75 | - | - | - | - |
| | Lactose monohydrat e (Tablettose 80) | - | - | - | - | 80.5 | 77.5 | 83.5 | 68.5 |
| Disintegr ant | Croscarmell ose sodium | 15 | 15 | 15 | 15 | 15 | 15 | 15 | 30 |
| Solubiliz | Sodium | 6 | 3 | - | 3 | 3 | 6 | - | - |
| er | lauryl sulfate (SLS) | | | | | | | | |
| | Poloxamer 407 | - | 10 | - | - | - | - | - | - |
| | Magnesium oxide | - | - | - | 20 | - | - | - | - |
| Lubrican t | Sodium stearyl fumarate | 2 | 2 | 2 | 2 | - | - | - | - |
| | Magnesium stearate | - | - | - | - | 1.5 | 1.5 | 1.5 | 1.5 |
| Average Weight (mg/formula) | | 370 | 380 | 370 | 390 | 300 | 300 | 300 | 300 |

### Dissolution Rate Evaluation of Example 4-7 and Comparative Examples 1-4

The dissolution rates of the formulations prepared in Example 4-7 (tablets) and Comparative Examples 1-4 (capsules) were evaluated. The dissolution rate results, along with the HLB values of the solubilizers, are shown in the table below. The 120-minute dissolution rate evaluation results for Example 4-7 are also shown in Figure 2.

**[Table 9]**

| | Examp le 4 010-1 | Example 5 014 | Exampl e 6 015 | Example 7 016 | Comparati Example GNT 200#3 | Comparati ve Example 2 GNT 200#4 | Comparati ve Example 3 GNT 200#1 | Comparati ve Example 4 GNT 200#2 |
|---|---|---|---|---|---|---|---|---|
| Solubiliz er (Q) | SLS (6 mg) | SLS (3 mg) & Poloxamer4 07 (10 mg) | No surfacta nt | Magnesiu m oxide (20 mg) | SLS (3mg) | SLS (6mg) | SLS (0mg) | SLS (0mg) |
| HLB value | 40 | 40 & 18~23 | N/A | Alkalizin g agent | 40 | 40 | 0 | 0 |
| Dissoluti on Rate | 10 min, 81.1% | 10 min, 73.7% | 10 min, 74.1% | 10 min, 82.7% | 15 min, 79.1% | 15 min, 77.0% | 15 min, 6.0% | 15 min, 10.5% |

As shown in the table above, when "1.62% SLS" was used as a solubilizer (Example 4), an increase in initial dissolution was observed compared to when "0.79% SLS and 2.63% Poloxamer 407" was used (Example 5) or the "surfactant-free" example. However, in Example 4, the increase in dissolution rate was not significant, even though the SLS content was increased 2.05-fold compared to Example 5.

Furthermore, the tablets according to the present invention exhibited faster dissolution rates than the capsule formulations of Comparative Examples 1-4. Considering that capsule formulations typically exhibit faster dissolution rates than tablets, the inclusion of D-mannitol and microcrystalline cellulose as diluents is thought to provide improved dissolution rates compared to formulations containing lactose monohydrate (e.g., Tablettose 80). Even with the same capsule formulation, Comparative Examples 1-2, which contained SLS, showed a marked increase in dissolution rate compared to Comparative Examples 3-4, which did not contain SLS. However, there was no significant difference in dissolution rate between 1% and 2% SLS, suggesting that the critical micelle concentration (CMC) of SLS is below 1%.

When magnesium oxide (MgO) was used (Example 7), an excellent dissolution rate of 82.7% was achieved in 10 minutes. However, when compressed to a target hardness of 7-9 kPa, the target hardness was not achieved. The appearance of the tablet changed to a bluish gray color during the short period between mixing and compression. The other formulations exhibited no problems with tableting. Moreover, in a compatibility study of crisdesalazine : MgO (1:1), when stability was evaluated under long-term storage conditions (25°C/60% relative humidity) or accelerated conditions (40°C/60% relative humidity) in a closed state for 13 weeks in a transparent glass vial, or closed for 7 weeks followed by open for 6 weeks, all mixtures showed an incompatible appearance change of turning gray, so although the dissolution was good, the formulation was not considered further.

### Comparative Stability Evaluation of Various Formulations

Formulations were prepared using methods similar to those used in the previous examples or comparative examples and their long-term storage stability was evaluated. The formulations for each formulation are listed in the table below.

**[Table 10]**

| Composition of the formulation | WO2020 136441A1 (Claim 5) | Batch No. | | | Batch No. | |
|---|---|---|---|---|---|---|
| | Capsule | Capsule | | | Tablet | |
| | Batch No. N/A | GPGF-1601 | GPGO-1601 | GPGT-1601 | C20003 | C20004 |
| Crisdesalazine | 1 to 1,000 mg | 50 mg (16.67%) | 100 mg (33.33%) | 200 mg (66.67%) | 100 mg (47.62%) | 200 mg (47.62%) |
| Lactose monohydrate | 60% | 233.5 mg (77.83%) | 180.50 mg (60.17%) | 80.5 mg (26.83%) | - | - |
| D-Mannitol (100SD) | - | - | - | - | 48.5 mg (23.10%) | 97 mg (23.10%) |
| Microcrystalline cellulose | - | - | - | - | 40 mg (19.05%) | 80 mg (19.05%) |
| Croscarmellose sodium | 5.0% | 15.0 mg (5.0%) | 15.00 (5.0%) | 15 mg (5.0%) | 7.5 mg (3.57%) | 15 mg (3.57%) |
| Magnesium stearate | 0.5% | 1.50 mg (0.5%) | 1.50 mg (0.5%) | 1.50 mg (0.5%) | - | - |
| Sodium stearyl fumarate | - | - | - | - | 2 mg (0.95%) | 4 mg (0.95%) |
| Sodium lauryl sulfate | 1.0% | - | 3.00 mg (1.0%) | 3.00 mg (1.0%) | 2 mg (0.95%) | 4 mg (0.95%) |
| Gelatin Capsule Shell | NA | Size 0 | Size 0 | Size 0 | NA | NA |
| Film Coating | NA | NA | NA | NA | 10 mg (4.76%) | 20 mg (4.76%) |
| Average weight | NA | 300mg | 300mg | 300mg | 210mg | 420mg |

The results of the long-term stability evaluation under conditions of 25 ± 2 °C and 60 ± 5% relative humidity are shown in the table below.

**[Table 11]**

| Composition of the formulation | WO2020 136441A1 (Claim 5) | Batch No. | | | Batch No. | |
|---|---|---|---|---|---|---|
| | Capsule | Capsule | | | Tablet | |
| | Batch No. N/A | GPGF-1601 | GPGO-1601 | GPGT-1601 | C20003 | C20004 |
| Initial | | | | | | |
| Maximum unknown impurity (%) | N/A | 0.09 | 0.08 | 0.07 | 0.02 | 0.02 |
| | | Passing criteria: ≤0.2% | | | Passing criteria: ≤0.15% | |
| Total Impurities (%) | N/A | 0.30 | 0.32 | 0.26 | 0.2 | 0.2 |
| | | Passing criteria: ≤0.8% | | | Passing criteria: ≤0.8% | |

| 18 months | | | | | | |
|---|---|---|---|---|---|---|
| Maximum unknown impurity (%) | N/A | 0.18 | 0.09 | 0.05 | 0.03 | 0.03 |
| | | Passing criteria: ≤0.2% | | | Passing criteria: ≤0.15% | |
| Total Impurities (%) | N/A | 0.46 | 0.39 | 0.31 | 0.16 | 0.16 |
| | | Passing criteria: ≤0.8% | | | Passing criteria: ≤0.8% | |

| 24 months | | | | | | |
|---|---|---|---|---|---|---|
| Maximum unknown impurity (%) | N/A | 0.18 | 0.13 | 0.14 | 0.02 | 0.02 |
| | | Passing criteria: ≤0.2% | | | Passing criteria: ≤0.15% | |
| Total Impurities (%) | N/A | 0.61 | 0.46 | 0.47 | 0.15 | 0.15 |
| | | Passing criteria: ≤0.8% | | | Passing criteria: ≤0.8% | |

| 30 months | | | | | | |
|---|---|---|---|---|---|---|
| Maximum unknown impurity (%) | N/A | 1.03 | 0.59 | 0.24 | 0.06 | 0.04 |
| | | Passing criteria: ≤0.2% | | | Passing criteria: ≤0.15% | |
| Total Impurities (%) | N/A | 1.27 | 0.74 | 0.35 | 0.3 | 0.3 |
| | | Passing criteria: ≤0.8% | | | Passing criteria: ≤0.8% | |

| 36 months | | | | | | |
|---|---|---|---|---|---|---|
| Maximum unknown impurity (%) | N/A | N/A | N/A | N/A | 0.04 | 0.04 |
| | | Passing criteria: ≤0.2% | | | Passing criteria: ≤0.15% | |
| Total Impurities (%) | N/A | N/A | N/A | N/A | 0.2 | 0.2 |
| | | Passing criteria: ≤0.8% | | | Passing criteria: ≤0.8% | |

| | | | | | | |
|---|---|---|---|---|---|---|
| N/A: Not Applied; % is average value of n=3 per formulation | | | | | | |

As can be seen from the results in the above table, the percentages of maximum unknown (individual) impurity and total impurities were significantly reduced by including D-mannitol and microcrystalline cellulose instead of lactose monohydrate as diluents. Crisdesalazine-100 mg tablets (Batch#C20003) and Crisdesalazine-200 mg tablets (Batch#C20004) provided in the present invention were very stable with little difference in the percentages of maximum unknown impurity and total impurities from the time of initial preparation to the subsequent 36 months (Table 11). That is, the percentage of maximum unknown impurity in Crisdesalazine-100 mg tablets (Batch#C20003) and Crisdesalazine-200 mg tablets (Batch#C20004) was 0.02% on average at the time of the initial, but was 0.04% on average after 36 months, and the percentage of total impurities was 0.2% on average at the time of the initial, but was 0.2% after 36 months, which was very stable (Table 11).

In addition, in a long-term stability test on pilot batches of Crisdesalazine-100 mg tablets (Batch#C20003) and Crisdesalazine-200 mg tablets (Batch#C20004), they were very stable with no difference in the percentage of maximum unknown impurity and total impurities from the time of preparation to 36 months thereafter (results not provided in Table 11 above). That is, in the pilot tablets (Table 3, composition of Example 1; n=3) of Crisdesalazine-100 mg tablets (Batch#C20003), the percentages of maximum unknown impurity were all at the BLOQ (<0.08%) level at the time of the initial, but were all at the BLOD (<0.025%) level even after 36 months, and the percentages of total impurities were 0.24%, 0.20%, and 0.20% (average 0.21%) at the time of the initial, but were 0.10%, 0.11%, and 0.12% (average 0.11%) even after 36 months, which were very stable. In the pilot tablets of Crisdesalazine-200 mg tablets (Batch#C20004) (Table 3, composition of Example 2; n=3), the percentages of maximum unknown impurity were all at the BLOQ (<0.08%) level at the time of the initial, but were all at the BLOD (<0.025%) level after 36 months. The percentages of total impurities were 0.24%, 0.17%, and 0.22% (average 0.21%) at the time of the initial, but were 0.10%, 0.10%, and 0.11% (average 0.10%) after 36 months, showing great stability. In the pilot tablets (n=3) of the Crisdesalazine-50 mg tablets (Table 3, composition of Example 3) provided in the present invention, the percentages of maximum unknown impurity were all at the BLOQ (<0.08%) level at the time of the initial, but were all at the BLOQ (<0.08%) level even after 36 months, and the percentages of total impurities were 0.25%, 0.21%, and 0.21% (average 0.22%) at the time of the initial, but were 0.23%, 0.22%, and 0.23% (average 0.23%) even after 36 months, which were also very stable (results not provided in Table 11 above).

There was no dose-dependent increase in impurities among the three doses of Crisdesalazine tablets (50 mg, 100 mg and 200 mg), probably because the proportions of each component in the formulation for both doses were the same.

In contrast, other formulations, namely, 1) "Formulation containing high proportion (77.83%) of lactose monohydrate but not 1% SLS (Batch #GPGF-1601)", 2) "Formulation containing medium proportion (60.17%) of lactose monohydrate and 1% SLS (Batch #GPGO-1601; WO2020136441A1, claim 5 has the same composition as Batch #GPGO-1601)", 3) "Formulation containing low proportion (26.83%) of lactose monohydrate and 1% SLS (Batch #GPGT-1601)", showed worse stability than the compositions provided by the present invention. That is, compared to the initial time at 24 months, the maximum unknown impurity of formulations 1), 2), and 3) increased by 2 times (0.18%), 1.6 times (0.13%), and 2 times (0.14%), respectively, and the total impurities deteriorated by 2.0 times (0.61%), 1.4 times (0.46%), and 1.8 times (0.47%), respectively. However, after 30 months, the maximum unknown impurity of formulations 1), 2), and 3) was 1.03%, 0.59%, and 0.24%, respectively, which did not meet the passing criteria (≤0.2%). The total impurities were 1.27%, 0.74%, and 0.35%, respectively. Formulation 1) did not meet the passing criteria (≤0.8%), while formulations 2) and 3) barely met it.

Therefore, the novel formulation according to the present invention [formulation containing mannitol and microcrystalline cellulose instead of lactose monohydrate and containing 1% SLS (batch # C20003 & C20004)] provides a formulation with improved long-term stability compared to previously published WO2020136441A1 claim 5 [formulation containing 1% SLS and 60.17% lactose monohydrate; batch # GPGT-1601] and unpublished formulations with different proportions of lactose monohydrate listed in the table above (batch # GPGF-1601, batch # GPGT-1601).

In the presence of 1% SLS, when the lactose monohydrate percentage decreased by 2.24-fold from 60.17% in the 100 mg capsules (batch # GPGO-1601) to 26.83% in the 200 mg capsules (batch # GPGO-1602), the maximum unknown impurity and total impurities at 30 months decreased by 2.46-fold and 2.11-fold, respectively. Therefore, the lactose monohydrate content is a major factor contributing to the decreased stability of crisdesalazine even in the presence of 1% SLS.

The proportion of lactose monohydrate (77.8% of the total amount) in the 50 mg capsule (#GPGF-1601) decreased 1.29-fold (60.17% of the total amount) in the 100 mg capsule (#GPGO-1601) and 2.90-fold (26.8% of the total amount) in the 200 mg capsule (#GPGT-1601). However, in the 30-month stability test, when compared to the 50 mg capsule (batch #GPGF-1601), the proportions of maximum unknown impurity and total impurities decreased 1.75-fold and 1.72-fold, respectively, in the 100 mg capsule (batch #GPGO-1601) and further decreased 4.29-fold and 3.62-fold, respectively, in the 200 mg capsule (batch #GPGT-1601). These results suggest that lactose content significantly affects the stability of crisdesalazine formulations. Furthermore, the magnitude of the decrease in the total impurities in both the 100 mg capsules (batch no. GPGO-1601) and 200 mg capsules (batch no. GPGT-1601) was greater than the decrease in lactose monohydrate content, suggesting that the 1% SLS content contributes to the stability of both the 100 mg and 200 mg capsules.

Based on the previously provided 3-month accelerated stability test results and the 36-month long-term storage stability test results for the "D-mannitol and microcrystalline cellulose" formulation, the maximum unknown impurity profile was established, allowing for a strict maximum unknown impurity criteria of ≤0.15% for the final drug product.

### Evaluation of Effect of Anionic Surfactants with HLB Above 8 on Dissolution Rate

Formulations similar to those in Comparative Examples 1 and 2 were prepared, with only the amounts of the active ingredient and SLS varied. Dissolution rates were evaluated at 15 minutes. The results are presented in the table below.

**[Table 12]**

| Ingredient | GNT 100#1 | GNT 100#2 | GNT 100#3 | GNT 200#1 | GNT 200#2 | GNT 200#3 | GNT 200#4 |
|---|---|---|---|---|---|---|---|
| Crisdesalazine (mg) | 100 | 100 | 100 | 200 | 200 | 200 | 200 |
| Sodium lauryl sulfate (SLS) (%) | N/A | N/A | 1 | N/A | N/A | 1 | 2 |

| Dissolution rate at 15 minutes | | | | | | | |
|---|---|---|---|---|---|---|---|
| Mean ± standard error | 27.8±6.8 | 50.4±2.9 | 86.8±3.2 | 6.0±1.0 | 10.5±1.5 | 79.1±6.5 | 77.0±19.6 |

As shown in the results in the table above, the presence or absence of SLS significantly affected the dissolution rate, and it was observed that SLS could enhance the solubility of crisdesalazine by increasing the intrinsic solubility of the formulation's internal components.

### Evaluation of Effect of Cationic Diluents on Dissolution Rate

Tablets were manufactured using the following formulation using a method similar to Example 1, and the dissolution rate was evaluated. The results are presented in the table below.

**[Table 13]**

| Intended Use | Ingredient | B#008 | B#009 | B#010 | B#011 | B#012 | B#013 |
|---|---|---|---|---|---|---|---|
| Active ingredient | Crisdesalazine | 200 | 200 | 200 | 200 | 200 | 200 |
| Diluent | D-Mannitol | 75 | 90 | 75 | 75 | - | 60 |
| | Microcrystalline cellulose | 75 | 90 | - | - | - | - |
| | Prosolve 90 | - | - | 75 | - | 75 | 90 |
| | Dibasic calcium phosphate | - | - | - | 75 | 75 | - |
| Disintegrant | Croscarmellose sodium | 15 | 15 | 15 | 15 | 15 | 15 |
| Solubilizer | Sodium lauryl sulfate | 3 | 3 | 3 | 3 | 3 | 3 |
| Lubricant | Sodium stearyl fumarate | 2 | 2 | 2 | 2 | 2 | 2 |
| Unit: weight (mg/tablet) | | 370 | 400 | 370 | 370 | 370 | 370 |

| Dissolution rate at 10 minutes in boric acid buffer solution at pH 8.6 | | | | | | | |
|---|---|---|---|---|---|---|---|
| Dissolution rate (%) | | 72.2 | 82.1 | 81.9 | 52 | 60.2 | 71.3 |

As shown in the table above, the B#011 and B#012 formulations containing dibasic calcium phosphate, a cationic diluent, showed significantly reduced dissolution rates and were also incompatible with crisdesalazine.

## Claims

1. A pharmaceutical composition comprising crisdesalazine or a pharmaceutically acceptable salt thereof as an active ingredient and a surfactant having an HLB (Hydrophilic and Lipophilic Balance) value of 8 or greater.

2. The pharmaceutical composition of claim 1, wherein the surfactant is sodium lauryl sulfate, polmoxamer-407, or a mixture thereof.

3. The pharmaceutical composition of claim 1 or 2, wherein the composition is substantially free of lactose.

4. The pharmaceutical composition of claim 3, wherein the composition comprises mannitol, microcrystalline cellulose, or a mixture thereof as a diluent.

5. The pharmaceutical composition of claim 1, wherein the composition comprises 5-70 wt% of crisdesalazine or a pharmaceutically acceptable salt thereof, 5-80 wt% of mannitol, 5-80 wt% of microcrystalline cellulose, 2-10 wt% of disintegrant, 0.1-10 wt% of sodium lauryl sulfate, and 0.1-10 wt% of lubricant, relative to the total weight of the composition.

6. The pharmaceutical composition of claim 5, wherein the composition comprises 5-50 wt% of crisdesalazine or a pharmaceutically acceptable salt thereof, 10-60 wt% of mannitol, 10-60 wt% of microcrystalline cellulose, 2-10 wt% of disintegrant, 0.5-5 wt% of sodium lauryl sulfate, and 0.5-5 wt% of a lubricant, relative to the total weight of the composition, and is in tablet form.

7. The pharmaceutical composition of claim 5 or 6, wherein the disintegrant is croscarmellose sodium.

8. The pharmaceutical composition of claim 5 or 6, wherein the lubricant is sodium stearyl fumarate.

9. The pharmaceutical composition of claim 1, wherein the composition has a pH of less than 7 when dispersed in 250 mL of purified water.

10. The pharmaceutical composition of claim 9, wherein the composition comprises an acidifying agent.

11. The pharmaceutical composition of claim 10, wherein the acidifying agent is fumaric acid, succinic acid, tartaric acid, or a mixture thereof.
